# EUROPÄISCHE PATENTSCHRIFT

(11) **EP 2 529 678 B1**
(45) Veröffentlichungstag und Bekanntmachung des Hinweises auf die Patenterteilung: **28.01.2015**
(21) Anmeldenummer: 11004439.3
(22) Anmeldetag: 31.05.2011
(51) Int. Cl.: A61B 17/22, A61B 17/225, A61B 17/92, A61N 7/00

(54) **Druckwellengerät zur Behandlung des menschlichen oder tierischen Körpers**
Pressure wave device for treating the human or animal body
Appareil à ondes de pression pour le traitement du corps humain ou animal

(43) Veröffentlichungstag der Anmeldung: 05.12.2012
(73) Patentinhaber: STORZ MEDICAL AG, 8274 Tägerwilen (CH)
(72) Erfinder: Marlinghaus, Ernst H., 8598 Bottighofen (CH); Katona, Josef, 78315 Radolfzell (DE); Schulz, Manfred, 8274 Tägerwilen/T (CH)
(74) Vertreter: Szynka, Dirk

(56) Entgegenhaltungen:
- EP-A1- 0 412 202
- EP-A1- 1 163 882
- EP-A1- 2 213 273
- EP-A2- 0 369 177
- WO-A2-94/17771
- DE-A1-102004 042 895
- DE-A1-102006 021 049
- DE-C1- 19 859 553
- US-A1- 2005 015 023

## Beschreibung

Diese Erfindung bezieht sich auf ein Druckwellengerät zur Behandlung des menschlichen oder tierischen Körpers durch mechanische Druckwellen.

Geräte zur Behandlung mit mechanischen Druckwellen sind an sich bekannt, insbesondere aus dem Bereich der Lithotripsie. Dort werden mit fokussierten mechanischen Druckwellen Körperkonkremente, insbesondere Steine im Körpergewebe, zertrümmert. In diesem Zusammenhang sind unterschiedliche Techniken zur Erzeugung der Druckwellen bekannt, etwa unter Verwendung von piezoelektrischen Elementen, durch Unterwasser-Funkenentladungen oder elektromagnetisch. Ein Beispiel für eine zur Lithotripsie geeignete elektromagnetische Druckwellenquelle findet sich in der EP 0 369 177 A2 der vorliegenden Anmelderin.

Neben den erwähnten Techniken sind auch Geräte entwickelt worden, die mechanische Druckwellen durch das Aufeinanderprallen eines beschleunigten Projektils und eines Prallkörpers erzeugen und mithilfe des Prallkörpers in Körpergewebe einkoppeln. Solche Geräte sind in der Lithotripsie verwendet worden, auch mit einem direkten Kontakt zwischen dem Prallkörper bzw. einer mit dem Prallkörper verbundenen Sonde und dem Stein, vgl. etwa die US 5,160,336. Ihre Anwendungen beinhalten aber auch andere Behandlungen von biologischen Körpersubstanzen, vgl. z. B. DE 197 25 477 A1. Insbesondere sind hier die Behandlung von Muskelerkrankungen und von Erkrankungen im Übergangsbereich zwischen Muskeln und Knochen zu nennen.

Dem Oberbegriff des Anspruchs 1 liegt die US 2005/0015023 A1 zugrunde, die insbesondere als eine erste von zwei Druckwellenquellen eine Zylinderquelle zeigt, die nach außen abstrahlt. Zusätzlich ist ein Parabolspiegel zur Fokussierung vorgesehen. Die zweite Druckwellenquelle ist elektrohydraulisch, elektromagnetisch oder piezoelektrisch.

Die DE 10 2006 021 049 A1 zeigt ebenfalls eine Vorrichtung mit zwei Druckwellenquellen, wobei eine erste eine Flachspulenanordnung mit Fokussiereinrichtung in

Form einer Linse aufweist und die zweite Druckwellenquelle in einer zentrischen Öffnung angeordnet ist.

Die EP 0 369 177 A2 betrachtet die Kombination einer zentral angeordneten Ultraschall-Ortungseinrichtung mit einer fokussierenden ersten Druckwellenquelle.

Die EP 2 213 273 A1 befasst sich im Detail mit einer ballistischen Druckwellenquelle.

Die DE 198 59 553 C1 sieht erneut eine fokussierende Druckwellenquelle mit einer Flachspule und einer Linse vor, wobei in einer zentrischen Öffnung eine Laserquelle angeordnet ist.

Die WO 94/17771 beschreibt im Ausführungsbeispiel aus Figur 2 eine zentral angeordnete Ultraschall-Einrichtung in einer darum herum aufgebauten fokussierenden Druckwellenquelle.

Die DE 10 2004 042 895 A1 befasst sich mit einer ballistischen Druckwellenquelle und einer Schutzkappe dafür.

Die EP 0 412 202 A1 zeigt erneut die Kombination einer fokussierenden Druckwellenquelle mit einer zentral angeordneten Ultraschall-Ortungseinrichtung.

Schließlich zeigt die EP 1 163 882 A1 einen ballistischen Lithotripter zur intrakorporalen Anwendung.

Der vorliegenden Erfindung liegt das technische Problem zugrunde, ein praxisgerechtes Druckwellengerät mit erweiterten Möglichkeiten anzugeben.

Das Problem wird gelöst durch ein Druckwellengerät zur Behandlung des menschlichen oder tierischen Körpers durch mechanische Druckwellen mit einer ersten Druckwellenquelle, die hohl ist und eine Abstrahlfläche für die erzeugten Druckwellen aufweist, die den Hohlraum umgibt und in Bezug auf diesen nach außen abstrahlen kann, und einem Reflektor zum Reflektieren und Ausrichten der abgestrahlten Druckwellen in den Körper, einer weiteren zweiten Druckwellenquelle, die zumindest teilweise in dem Hohlraum der ersten Druckwellenquelle angeordnet ist und Druckwellen in Bezug auf den Hohlraum in Längsrichtung abstrahlen kann, dadurch gekennzeichnet, dass die zweite Druckwellenquelle eine ballistische Druckwellenquelle ist, bei der ein Projektil beschleunigt werden kann und zu einem Aufprall zur Erzeugung der Druckwelle ausgelegt ist, und dass die erste Druckwellenquelle nicht-fokussierend ausgelegt ist.

Die Grundidee der Erfindung liegt darin, in einem Gerät zwei unterschiedliche Druckwellenquellen zu kombinieren und dabei eine hohle Konstruktion der ersten Druckwellenquelle zu verwenden. In dem Hohlraum lässt sich, zumindest teilweise, die zweite Druckwellenquelle unterbringen. Die erste Druckwellenquelle verfügt über eine Abstrahlfläche mit nach außen gerichteter Abstrahlrichtung, insbesondere radial bei einer bevorzugten zylindrischen oder rotationssymmetrischen Abstrahlfläche. Die zweite Druckwellenquelle strahlt im Wesentlichen bzw. schwerpunktmäßig in einer Längsrichtung des Hohlraums ab, insbesondere axial. Damit können in einer baulich günstigen und kompakten Weise unterschiedliche Konstruktionen von Druckwellenquellen kombiniert werden, die sich mit geringen oder ohne Unterbrechungen nacheinander oder sogar gleichzeitig am Patienten anwenden lassen.

Für die erste Druckwellenquelle kommen insbesondere und bevorzugt Hohlkonstruktionen entsprechend der bereits zitierten EP 0 369 177 A2 in Betracht, auf die diesbezüglich vollständig verwiesen wird. Sie zeigt insbesondere rotationssymmetrische Körper als Druckwellenquelle mit radial abstrahlenden Mantelflächen, wobei z. B. Kreiszylinder oder Kegelstümpfe in Betracht kommen. Die Abstrahlung kann aufgrund einer elektromagnetischen Druckwellenerzeugung vonstattengehen, aber auch mit piezoelektrischer Grundlage. Zusätzlich ist ein Reflektor zum Ausrichten durch Reflexion der abgestrahlten Druckwellen der ersten Druckwellenquelle vorgesehen. Die nach außen abgestrahlten Druckwellen werden durch den Reflektor umgelenkt und ausgerichtet und können dadurch insbesondere richtungsmäßig ähnlich oder schwerpunktmäßig gleich mit den Druckwellen der zweiten Druckwellenquelle angewendet werden.

Als zweite Druckwellenquelle wird eine längliche und längs abstrahlende Konstruktion gewählt, nämlich eine ballistische Konstruktion mit einem beschleunigten Projektil, das vorzugsweise auf einen Prallkörper auftrifft und damit eine Druckwelle erzeugt. Hierzu kann auf die bereits zitierte DE 197 25 477 A1 verwiesen werden. Der grundsätzliche Aufbau solcher ballistischer Druckwellenquellen ist dem Fachmann bekannt.

Im vorliegenden Zusammenhang sind allerdings verschiedene Merkmale dieses Standes der Technik nicht zwingend. Zunächst ist es nicht zwingend, dass ein Prallkörper zum Aufprall des Projektils verfügbar ist. Bei geeignet gewählten Projektilmaterialien und/oder -massen und -geschwindigkeiten ist es durchaus denkbar, das Projektil auch unmittelbar auf den Körper bzw. eine auf diesen aufgelegte Membran (vgl. Ausführungsbeispiel) aiufprallen zu lassen. In diesem Zusammenhang kann auch verwiesen werden auf die WO 94/17771, die Anwendungsmöglichkeiten hierfür beschreibt, aber auch für "klassische" Druckwellenquellen etwa mit Funkenentladungen und Reflektor. Ein Prallkörper ist allerdings im vorliegenden Zusammenhang bevorzugt.

Genauso wenig ist das Projektil selbst zwingend. Grundsätzlich kann ein Druckwellenpuls auch erzeugt werden z. B. durch Auftreffenlassen eines Druckluftpulses auf einen solchen Prallkörper, vgl. etwa die DE 20 2007 007 921 U1 der vorliegenden Anmelderin. Schließlich ist es nicht zwingend, einen pneumatischen Antrieb zur Beschleunigung eines Projektils zu verwenden. Hier sind auch andere Möglichkeiten, insbesondere elektrische oder elektromagnetische Beschleunigungsvorrichtungen, denkbar. Allerdings ist im vorliegenden Zusammenhang ein pneumatischer Antrieb und, insbesondere in Verbindung damit, ein Projektil bevorzugt.

Wenn nun z. B. eine elektromagnetische zylindrische erste Druckwellenquelle mit einer in dem Hohlraum angeordneten ballistischen zweiten Druckwellenquelle kombiniert wird, kann mit ein und demselben Gerät am Patienten gearbeitet werden, möglicherweise auch gleichzeitig, jedenfalls mit erheblicher Zeitersparnis. Z. B. könnte bei der Indikation eines sog. Tennisellenbogens eine Muskel- oder Muskelansatzbehandlung mit der zweiten Druckwellenquelle und zusätzlich eine Knochenbehandlung mit der ersten Druckwellenquelle vorgenommen werden. Dazu ist die erste Druckwellenquelle erfindungsgemäß nicht-fokussierend ausgelegt. In vielen Fällen wird die erste Druckwellenquelle Druckwellen mit steileren Anstiegsflanken und/oder höheren Druckamplituden erzeugen. Außerdem kann sie weiter in den zu behandelnden Körper hineinreichen und damit tiefer wirken. Beispielsweise kann bei Indikationen wie der Cellulitis eine oberflächennahe Behandlung mit der zweiten Druckwellenquelle kombiniert werden mit einer tiefer reichenden Behandlung mit der ersten Druckwellenquelle.

Erfindungsgemäß können die beiden Druckwellenquellen so ausgelegt sein, dass ihr Betrieb voneinander unabhängig erfolgen kann, also beispielsweise mit der ersten Druckwellenquelle und dann ergänzend nach Abschluss dieser Behandlung eine weitere Behandlung mit der zweiten Druckwellenquelle erfolgt oder umgekehrt. Dann liegt der Vorteil der Erfindung in der baulichen Vereinheitlichung und in der Praktikabilität des direkten Übergangs von der einen auf die andere Behandlung (und danach möglicherweise wieder zurück etc.). Allerdings können die beiden Druckwellenquellen vorteilhafterweise auch parallel betrieben werden, und zwar synchron oder auch asynchron. Mit "synchron" ist gemeint, dass die erste und die zweite Druckwellenquelle hinsichtlich der Erzeugung der einzelnen Druckwellenpulse in einer festen zeitlichen Beziehung zueinander stehen, wobei die Druckwellenpulse insbesondere auch gleichzeitig abgegeben werden können. Dabei können die Wiederhotfrequenzen gleich sein (also nur gleichzeitige Pulse abgegeben werden) oder auch die Wiederholfrequenz der einen Druckwellenquelle ein ganzzahliges Vielfaches der Wiederholfrequenz der anderen sein (sodass also nur bestimmte Druckwellenpulse der einen gleichzeitig mit Druckwellenpulsen der anderen erfolgen). Ein solcher synchroner Betrieb ist auch mit fester Phasenbeziehung, also mit gleichbleibendem Zeitversatz, zwischen den Druckwellenpulsen möglich. In Betracht gezogen wird aber auch ein asynchroner paralleler Betrieb, bei dem hinsichtlich Frequenz und Phasenbeziehung keine feste Beziehung bzw. kein einfacher Zusammenhang besteht. Das erfindungsgemäße Druckwellengerät kann für verschiedene dieser Betriebsarten ausgelegt sein oder auch nur für eine bestimmte davon.

Wie schon erwähnt ist die Abstrahlfläche der ersten Druckwellenquelle vorzugsweise rotationssymmetrisch, insbesondere kreiszylindrisch. Die elektromagnetische Druckwellenquelle weist dazu, wie in der zitierten Schrift beschrieben, vorzugsweise eine Flachspule und eine Membran auf. Der zugehörige Reflektor kann in Bezug auf die Symmetrieachse ebenfalls symmetrisch sein, wobei er nicht zwingend rotationssymmetrisch sein muss, vgl. etwa die in der erwähnten EP 0 369 177 A2 erwähnten elliptischen Querschnittsformen des Reflektors quer zur Symmetrieachse. Auch andere mehrzählige Symmetrien sind denkbar.

Der Reflektor kann zusammen mit einer Membran ein Volumen begrenzen, in dem ein flüssiges, d. h. im vorliegenden Zusammenhang fließfähiges, Koppelmedium eingeschlossen ist, z. B. Wasser. Das Koppelmedium hat vorteilhafterweise ähnliche Impedanzen hinsichtlich der erzeugten Druckwellen im Körpergewebe. Es kann die Membran etwas vorwölben, wodurch diese bezüglich der Längsrichtung des Hohlraums etwas über die Vorderseite der zweiten Druckwellenquelle vorgelagert liegen kann und/oder gegenüber der Vorderkante des Reflektors vorgelagert liegen kann. Insbesondere kann die zweite Druckwellenquelle längs des Hohlraums verschiebbar sein, sodass je nach Bedarf weiter auf den Körper zu geschoben oder zurückgefahren werden kann.

Insbesondere kann durch die vorgelagerte Membran ein "weicher" und sich in der Form anpassender Andruck des Gesamtgeräts auf dem Körper erzeugt werden. Dabei schmiegt sich die Membran an die Körperfläche zumindest teilweise an und sorgt für eine großflächige gute Anlage mit guter Druckwellenankopplung. Hierbei kann es zur Anwendung der zweiten Druckwelle gewünscht sein, dass diese direkt auf dem Körper (unter Zwischenschalten der Membran) aufliegt, was durch entsprechenden Andruck und/oder Vorschieben der zweiten Druckwellenquelle erreicht werden kann. Andererseits kann, z. B. wenn nur die erste Druckwellenquelle benutzt wird, auch dafür gesorgt werden, dass die zweite Druckwellenquelle zurückgeschoben bzw. durch geringeren Andruck nicht in Kontakt mit der Körperoberfläche ist.

Eine bevorzugte Ausgestaltung der Erfindung wird im folgenden Ausführungsbeispiel näher erläutert, wobei die einzelnen Merkmale auch in anderen Kombinationen erfindungswesentlich sein können. Ferner wird darauf hingewiesen, dass sich die Erfindung auch auf eine Verwendung der jeweiligen Druckwellenquellen für das kombinierte Druckwellengerät bezieht. Die vorstehende und die nachfolgende Offenbarung sind für alle Erfindungskategorien von Bedeutung.
- Figur 1: zeigt eine ballistische Druckwellenquelle im Längsschnitt, die in einer erfindungsgemäßen Vorrichtung gemäß Figur 2 eingesetzt werden kann.
- Figur 2: zeigt ebenfalls Im Längsschnitt einen wesentlichen Teil eines erfindungsgemäßen Druckwellengeräts mit einer etwas von Figur 1 abweichenden ballistischen Druckwellenquelle und einer weiteren elektromagnetischen zylindrischen Druckwellenquelle.

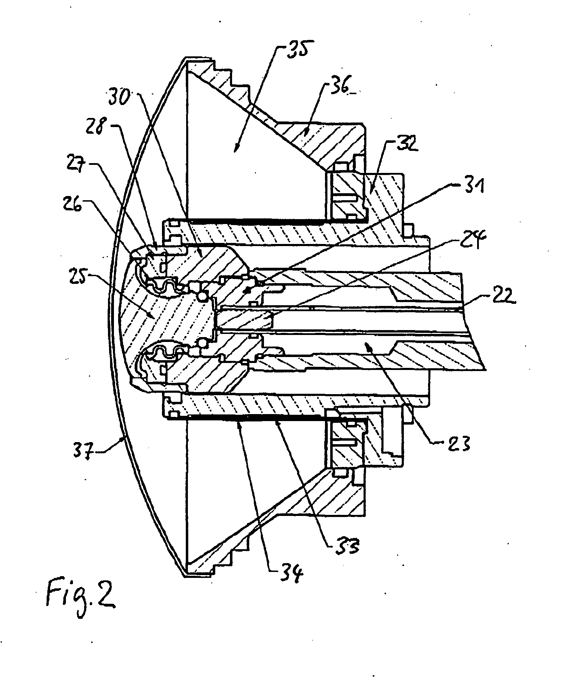

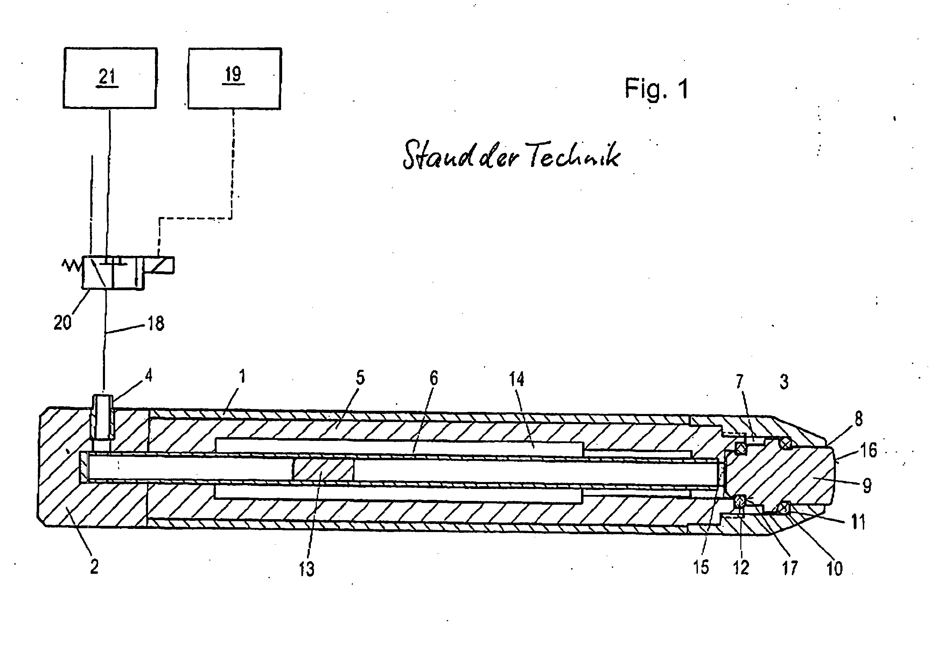

Zunächst wird anhand Figur 1 der grundsätzliche Aufbau eines ballistischen Druckwellengeräts erläutert, das im vorliegenden Zusammenhang als zweite Druckwellenquelle dient. Auf die erste Druckwellenquelle wird daraufhin anhand Figur 2 eingegangen. Gemäß Figur 1 bildet ein Rohrstück eine Außenhülse 1, die von einer in der Anwendung körperfemen Zuluftkappe 2 und einer in der Anwendung körperzugewandten Applikatorkappe 3 jeweils endseitig abgeschlossen ist.

Die Zuluftkappe 2 enthält einen Druckluftanschluss 4 für eine pneumatische Versorgung. In an sich bekannter Weise ist an diesen Druckluftanschluss 4 über eine pneumatische Versorgungsleitung ein von einer Ansteuereinheit 19 gesteuertes Ventil 20, insbesondere Magnetventil, angeschlossen, das in einem gleichbleibenden iterativen Takt zwischen etwa 1 Hz und 50 Hz Druckluftpulse über den Druckluftanschluss einkoppelt.

Die Vorrichtung ist über die erwähnte Pneumatikleitung 18 an eine Basisstation mit der Ansteuereinheit 19 und dem Kompressor 21 angeschlossen und kann auf den Patienten manuell aufgesetzt werden.

In der Außenhülse 1 ist über einen Einsatz 5 ein Führungsrohr 6 gehalten, dessen bei der Anwendung körperfemes Ende in der Zuluftkappe 2 endet und dort mit dem Druckluftanschluss 4 kommuniziert. Das in der Anwendung körperseitige Ende des Führungsrohres 6 endet in einem Teil des Einsatzes 5, der in die Applikatorkappe 3 hineinragt, und zwar kurz vor dem dortigen Ende des Einsatzes 5 und einem Innenraum 7 in der Applikatorkappe 3.

In dem Innenraum 7. der mit zwei radialen Schultern in eine in der Anwendung körperseitige Applikatoröffnung 8 übergeht, ist ein Prallkörper 9 aufgenommen. Dieser stützt sich über einen O-Ring 10 aus einem Elastomer an einer der radialen Schultern ab und weist hierzu einen Flansch 11 auf. Ein zur körperfemen Seite gerichtetes Ende 15 des Prallkörpers 9 stützt sich über einen weiteren O-Ring 12 an dem Einsatz 5 ab, und zwar an einer das bereits erwähnte Ende des Einsatzes 5 umgebenden Stimfläche. Dabei liegt der O-Ring 12 zwischen dieser Stimfläche und einem Flansch 17 bzw. einer Schulter des Prallkörpers 9. Die Applikatoröffnung 8 dient dabei zu einer in der Längsrichtung verschiebbaren Führung des Prallkörpers 9 und fixiert diesen quer zur Längsrichtung. Die Axialverschiebbarkeit ist durch die Nachgiebigkeit der Elastomerringe 10 und 12 begrenzt und liegt bei in Luft betriebener Vorrichtung relativ zur Restvorrichtung z. B. deutlich über 0.6 mm.

In dem an den Prallkörper 9 angrenzenden Bereich des Führungsrohres 6 ist ein in Figur 1 mit dem Prallkörper 9 in Kontakt stehendes Projektil 13 eingesetzt. Dieses passt (in Bezug auf das Führungsrohr und die im Wesentlichen zylindrische Geometrie des Projektils 13) radial mit geringem Spiel. Das Projektil 13 kann durch Druckunterschiede der Luftsäule in dem Führungsrohr 6 vor und hinter ihm (d. h. in Figur 1 rechts und links des Projektils 13) in dem Führungsrohr hin- und herbewegt werden und insbesondere auf den Prallkörper 9 zu beschleunigt werden. Hierzu wird es aus einer Ausgangsposition (nicht gezeigt) in Figur 1 links durch einen Druckluftstoß durch den Druckluftanschluss 4 beschleunigt und trifft mit seiner dem Prallkörper 9 zugewandten Frontfläche (in Figur 1 der Übersichtlichkeit halber nicht bezeichnet) auf den Prallkörper 9 auf.

Die Rückbewegung des Projektils 13 erfolgt zusätzlich zu einem Zurückprallen nach der Kollision durch ein Rückströmen der Luft aus einer das Führungsrohr 6 innerhalb des Einsatzes 5 umgebenden Staukammer 14. In diese wird die Luft bei der Beschleunigung des Projektils 13 in Richtung zu dem Prallkörper 9 verdrängt und damit dort komprimiert. Wenn das Magnetventil 20 in der Pneumatikzuleitung 18 des Druckluftanschlusses 4 den Druck wegschaltet, wird das Projektil 13 damit in die Ausgangsstellung zurückbewegt. Dies kann natürlich auch durch eine zusätzliche oder alternative Druckbeaufschlagung der Staukammer 14 oder eines anderen Luftvolumens körperseitig von dem Projektil 13 erfolgen. Das in der Anwendung körperferne Ende des Führungsrohres 6 endet in einem Magnethalter 17 für das Projektil 13.

Der Prallkörper 9 hat eine rotationssymmetrische angenäherte Zylinderform und ist in axialer Richtung durch die Eintrittsfläche 15 und die etwas konvexe Austrittsfläche 16 begrenzt. Der Außenmantel weist die bereits beschriebenen flanschartigen Strukturen 11 und 17 auf, die Anlageschultem für die O-Ringe 10 und 12 bilden. Im Übrigen ist ein austrittsseitiger Teil der Zylindergeometrie mit konstantem Radius gestaltet und damit in der Öffnung 8 axial verschiebbar.

Bislang wurde Stand der Technik beschrieben. Figur 2 zeigt abweichend davon die Erfindung. In Figur 2 ist zunächst mit 22 ein Führungsrohr wie das Führungsrohr 6 aus Figur 1 dargestellt, wobei in Figur 2 der dem linken Bereich der Figur 1 entsprechende Abschnitt mit der Darstellung der pneumatischen Beschleunigungseinrichtung mit Ausnahme des letzten Führungsrohrbereichs und der mit 23 bezeichneten Staukammer, vgl. 14 in Figur 1, weggelassen ist. In dem Führungsrohr 22 ist ein Projektil 24 gezeigt, das dem Projektil 13 aus Figur 1 entspricht. Dieses ist am linken Ende des Führungsrohres 22 angelangt und berührt mit seiner linken Frontfläche die rechte Eintrittsfläche eines Prallkörpers 25, der in seiner Funktion weit gehend dem Prallkörper 9 aus Figur 1 entspricht. Die in Figur 2 linke Austrittsfläche dieses Prallkörpers, vgl. die Fläche 16 in Figur 1, ist im Durchmesser erheblich vergrößert gegenüber der Eintrittsfläche und leicht konvex gekrümmt. Der Prallkörper 25 ist in seinem rechten Berelch an einem nicht näher bezeichneten O-Ring gehalten, der im Wesentlichen dem O-Ring 12 aus Figur 1 entspricht. Etwas weiter links davon stützt sich an einer Außenrille des Prallkörpers 25 ein elastischer Kunststoffwellschlauch 26 ab, dessen anderes, in Figur 2 linkes Ende sich an einem inneren Kragen eines rotationssymmetrischen Montagerings 27 abstützt. Der elastische Wellschlauch 26 hat die gleiche Funktion wie der elastische O-Ring 10 aus Figur 1, erlaubt aber einen sehr viel größeren Hub. Bei dieser Ausgestaltung ist also im Unterschied zu Figur 1 eine relativ große makroskopische Schwerpunktbewegung des Prallkörpers 25 möglich.

Der erwähnte Montagering 27 ist nach außen von einer im Querschnitt flach erscheinenden Montagemutter 28 gehalten, deren in Figur 2 linke Innenmantelfläche den vordersten Bereich des Prallkörpers 25 führt, Die Montagemutter 28 lst im Übrigen mit ihrem in Figur 2 rechten Bereich aufgeschraubt auf einen weiteren Montagekörper 30, der seinerseits aufgeschraubt ist auf einen Inneren Montagekörper 31, der das in Figur 2 linke Ende des Führungsrohres 22 umgibt und an dessen Ende mit etwas geringerem freien Radius in die Öffnung des Führungsrohres 22 eingreift. Hierdurch und durch die leichte konische Verjüngung des Projektils 24 wird erreicht, dass das Projektil zwar an die Eintrittsfläche des Prallkörpers anschlagen kann, aber bei demontiertem Prallkörper das Führungsrohr 22 nicht weiter verlassen kann. Es handelt sich also um eine Sicherung.

Die Teile 28 bis 31 haben zusammen eine vergleichbare Funktion wie die den Prallkörper 9 in Figur 1 haltende Applikatorkappe 3. Insgesamt dient die Mehrteiligkeit zur Montage und Demontage des Prallkörpers 25 und des Wellschlauchs 26 bei gleichzeitiger Abdichtung (durch Dichtungen in den eingezeichneten rechteckigen Hohlräumen). Das Beispiel veranschaulicht die Möglichkeit der Verwendung eines im Durchmesser und in der Länge gegenüber Figur 1 deutlich vergrößerten Prallkörpers 25 mit abweichendem Aufbau. Umgekehrt gilt natürlich auch, dass sich auch eine Vorrichtung wie aus Figur 1 verwenden lassen würde.

In jedem Fall ist die zweite Druckwellenquelle. nämlich das bisher behandelte ballistische Druckwellengerät, in einen Spulenhalter 32 mit im linken Bereich im Wesentlichen hohlzylindrischer Form und rechts erkennbarem Kragen eingesetzt. Dabei ist die ballistische Druckwellenquelle in einer nicht näher ausgeführten Form horizontal verschieblich und in Figur 2 in einer relativ weit vorgeschobenen Position gezeigt. Sie kann also nach rechts zurückgezogen werden.

An der Außenmantelfläche des hohlzylindrischen Teils des Spulenhalters 32 ist eine flache Spulenwicklung 33 einer Zylinderspule aufgebracht, was in der bereits mehrfach zitierten EP 0 369 177 A2 näher dargestellt ist. Diese ist von einer Membran 34 als kreiszylindrische Abstrahlfläche umgeben, die durch stoßartige Magnetfelder der Flachspule 33 und die daraus resultierende abstoßende Wechselwirkung zwischen induzierten Strömen in der Membran 34 und diesen Feldern stoßartig nach außen getrieben werden kann.

Diese Zylinderspulenkonstruktion befindet sich in einem Wasservolumen 35, das eingeschlossen ist zwischen einem in diesem Fall konischen Reflektor 36 und einer an dessen linkem vordersten Rand angebrachten und nach links ausgewölbten Membran 37. Der Wasservorrat 35 ist in einer in der zitierten Voranmeldung bereits erwähnten Form in nicht gezeigter Weise an ein Ausgleichsvolumen angeschlossen und kann daher bei einem gewissen vorgegebenen Innendruck durch Bewegung in der Membran 37 im Volumen verändert werden.

Hinsichtlich der Funktionsweise und des Betriebs der Zylinderspule wird auf den zitierten Stand der Technik verwiesen. Man erkennt leicht, dass die kreiszylindrische Abstrahlfläche, nämlich die Membran 34, radial nach außen abstrahlt und diese Welle durch den Reflektor 36 nach links umgelenkt wird.

Die in Figur 2 zusätzlich zu dem ballistischen Druckwellengerät enthaltenen Bauteile bilden mit diesem gemeinsam eine mit der Hand handhabbare Einheit, die in ähnlicher Weise wie im Zusammenhang mit Figur 1 erwähnt über eine Versorgungsleitung, in diesem Fall mit pneumatischen und elektrischen Leitungen, an eine Basisstation angeschlossen ist.

Das Gesamtgerät kann mit der Membran 37 auf die Hautoberfläche des zu behandelnden Körpers aufgesetzt werden. Bei vorgeschobenem ballistischem Druckwellengerät ergibt sich dabei in der Mitte der Membran 37 ein durch diese vermittelter quasi direkter Kontakt zwischen der Austrittsfläche des Prallkörpers 25 und der Körperoberfläche, sodass in der bereits bekannten Weise ballistisch erzeugte Druckwellen eingekoppelt werden können. Die Membran 37 stört dabei nicht wesentlich; sie ist vielmehr aus impedanzmäßig geeignetem Material und dünn genug gefertigt, um keine wesentlichen Verluste zu bewirken. Grundsätzlich könnten die Druckwellen sogar bei verbleibenden Wasserschichten zwischen Membran 37 und Austrittsfläche des Prallkörpers 25 eingekoppelt werden.

Gleichzeitig oder alternativ können wesentlich intensivere Druckwellen elektromagnetisch erzeugt und durch den Reflektor über die Membran in den Körper eingebracht werden. Dazu wird vorteilhafterweise das ballistische Druckwellengerät etwas zurückgezogen, sodass die Membran möglichst großflächig am Körper anliegen kann, vgl. die Figuren 2 bis 4 der EP 0 369 177 A2. Allerdings ist im vorliegenden Zusammenhang weniger an Lithotripsie gedacht sondern eher an die kombinierte Behandlung von Krankheitsbildern wie Tennisellenbogen oder Cellulitis, die.bereits erwähnt wurden, oder die Behandlung von orthopädischen Weichgewebe-Krankheitsbildern oder von Sehnenerkrankungen. Diese Angaben sind aber nicht einschränkend.

## Patentansprüche

1. Druckwellengerät zur Behandlung des menschlichen oder tierischen Körpers durch mechanische Druckwellen mit
einer ersten Druckwellenquelle (32-34), die hohl ist und eine Abstrahlfläche (34) für die erzeugten Druckwellen aufweist, die den Hohlraum umgibt und in Bezug auf diesen nach außen abstrahlen kann,
einem Reflektor (36) zum Reflektieren und Ausrichten der abgestrahlten Druckwellen in den Körper,
einer weiteren zweiten Druckwellenquelle (22-30), die zumindest teilweise in dem Hohlraum der ersten Druckwellenquelle (32-34) angeordnet ist und Druckwellen in Bezug auf den Hohlraum in Längsrichtung abstrahlen kann, **dadurch gekennzeichnet, dass** die zweite Druckwellenquelle (22-30) eine ballistische Druckwellenquelle ist, bei der ein Projektil (24) beschleunigt werden kann und zu einem Aufprall zur Erzeugung der Druckwelle ausgelegt ist,
und dass die erste Druckwellenquelle (32-34) nicht-fokussierend ausgelegt ist.

2. Druckwellengerät nach Anspruch 1, bei der die zweite Druckwellenquelle (22-30) einen Prallkörper (25) zur Erzeugung der Druckwelle durch einen Aufprall darauf aufweist.

3. Druckwellengerät nach einem der vorstehenden Ansprüche, bei der die zweite Druckwellenquelle (22-30) eine pneumatische Vorrichtung (18-21) zur Erzeugung der Druckwelle aufweist.

4. Druckwellengerät nach einem der vorstehenden Ansprüche, bei der die Abstrahlfläche (34) der ersten Druckwellenquelle (32-34) rotationssymmetrisch um eine durch den Hohlraum verlaufende Achse ist und der Reflektor (36) symmetrisch in Bezug auf diese Achse ist.

5. Druckwellengerät nach Anspruch 4, bei dem der Reflektor (36) konisch ist.

6. Druckwellengerät nach einem der vorstehenden Ansprüche, bei dem eine Membran (37) vorgesehen ist, den Reflektor (36) zur Seite des zu behandelnden Körpers hin abdeckt und zwischen sich und dem Reflektor (36) ein flüssiges Koppelmedium (35) einschließt.

7. Druckwellengerät nach Anspruch 6, bei dem sich die Membran (37) vor die Vorderkante des Reflektors (36) vorwölbt und dabei einer dem Körper zugewandten Vorderseite der zweiten Druckwellenquelle (22-30) vorgelagert sein kann.

8. Druckwellengerät nach einem der vorstehenden Ansprüche, bei dem die zweite Druckwellenquelle (22-30) längs des Hohlraums verschiebbar ist.

9. Druckwellengerät nach einem der vorstehenden Ansprüche, bei dem die erste Druckwellenquelle (32-34) elektromagnetisch funktioniert und eine den Hohlraum umgebende Flachspule (33) sowie eine Membran (34) aufweist.

## Claims

1. A shock wave apparatus for treating the human or animal body by means of mechanical shock waves, having
a first shock wave source (32-34) being hollow and having a radiating surface (34) for the shock waves produced which surrounds the hollow space and which can radiate to the outside in relation to the hollow space,
a reflector (36) for reflecting and directing the radiated shock waves into the body,
a further second shock wave source (22-30) being arranged within the hollow space of the first shock wave source (32-34) at least partially and being able to radiate shock waves in the longitudinal direction in relation to the hollow space,
**characterized in that** the second shock wave source (22-30) is a ballistic shock wave source wherein a projectile (24) can be accelerated and is adapted to a collision for producing the shock wave
and wherein the first shock wave source (32-34) is adapted to be non-focusing.

2. The shock wave apparatus of claim 1 wherein the second shock wave source (22-30) comprises a collision body (25) for producing the shock wave by a collision thereon.

3. The shock wave apparatus of one of the preceding claims wherein the second shock wave source (22-30) comprises a pneumatic device (18-21) for producing the shock wave.

4. The shock wave apparatus of one of the preceding claims wherein the radiating surface (34) of the first shock wave source (32-34) is rotationally symmetric around an axis through the hollow space and the reflector (36) is symmetric with regard to this axis.

5. The shock wave apparatus of claim 4 wherein the reflector (36) is conical.

6. The shock wave apparatus of one of the preceding claims wherein a membrane (37) is provided covering the reflector (36) to the side of the body to be treated and enclosing a liquid coupling medium (35) between itself and the reflector (36).

7. The shock wave apparatus of claim 6 wherein the membrane (37) bulges forwardly in front of the front edge of the reflector (36) and can be arranged in front of a front side of the second shock wave source (22-30) facing the body.

8. The shock wave apparatus of one of the preceding claims wherein the second shock wave source (22-30) is displaceable longitudinally along the hollow space.

9. The shock wave apparatus of one of the preceding claims wherein the first shock wave source (32-34) works electromagnetically and comprises a flat coil (33) surrounding the hollow space and a membrane (34).

## Revendications

1. Appareil à ondes de pression pour le traitement du corps humain ou animal par ondes de pression mécaniques, comportant
une première source d'ondes de pression (32-34) qui est creuse et qui présente une surface rayonnante (34) destinée aux ondes de pression produites, laquelle entoure la cavité et peut rayonner vers l'extérieur par rapport à ladite cavité,
un réflecteur (36) destiné à réfléchir et à diriger dans le corps les ondes de pression rayonnées,
une deuxième source d'ondes de pression (22-30) disposée au moins en partie dans la cavité de la première source d'ondes de pression (32-34) et susceptible de rayonner les ondes de pression dans le sens longitudinal par rapport à la cavité,
**caractérisé en ce que** la deuxième source d'ondes de pression (22-30) est une source d'ondes de pression balistiques dans laquelle un projectile (24) peut subir une accélération pour entraîner un impact permettant de produire l'onde de pression,
et **en ce que** la première source d'ondes de pression (32-34) est conçue pour être non focalisante.

2. Appareil à ondes de pression selon la revendication 1, dans lequel la deuxième source d'ondes de pression (22-30) présente un corps d'impact (25) destiné à produire l'onde de pression sous l'effet d'un impact exercé sur celui-ci.

3. Appareil à ondes de pression selon l'une des revendications précédentes, dans lequel la deuxième source d'ondes de pression (22-30) présente un dispositif pneumatique (18-21) destiné à produire l'onde de pression.

4. Appareil à ondes de pression selon l'une des revendications précédentes, dans lequel la surface rayonnante (34) de la première source d'ondes de pression (32-34) est symétrique en rotation autour d'un axe traversant la cavité et le réflecteur (36) est symétrique par rapport à cet axe.

5. Appareil à ondes de pression selon la revendication 4, dans lequel le réflecteur (36) est conique.

6. Appareil à ondes de pression selon l'une des revendications précédentes, dans lequel il est prévu une membrane (37) qui couvre le réflecteur (36) sur le côté tourné vers le corps à traiter et dans lequel un milieu de couplage fluide (35) est contenu entre ladite membrane et le réflecteur (36).

7. Appareil à ondes de pression selon la revendication 6, dans lequel la membrane (37) est renflée devant le bord avant du réflecteur (36) et peut ainsi précéder un côté avant de la deuxième source d'ondes de pression (22-30) qui est tourné vers le corps.

8. Appareil à ondes de pression selon l'une des revendications précédentes, dans lequel la deuxième source d'ondes de pression (22-30) est déplaçable le long de la cavité.

9. Appareil à ondes de pression selon l'une des revendications précédentes, dans lequel la première source d'ondes de pression (32-34) fonctionne par voie électromagnétique et présente une bobine plate (33), qui entoure la cavité, ainsi qu'une membrane (34).
